# EUROPEAN PATENT SPECIFICATION

(11) **EP 3 572 524 B1**
(45) Date of publication and mention of the grant of the patent: **28.01.2026**
(21) Application number: 17892276.1
(22) Date of filing: 28.12.2017
(51) Int. Cl.: C12Q 1/6881

(54) **METHOD FOR EVALUATING CELL DIFFERENTIATION STATE**
VERFAHREN ZUR EVALUIERUNG DES ZELLDIFFERENZIERUNGSZUSTANDS
PROCÉDÉ D'ÉVALUATION DE L'ÉTAT DE DIFFÉRENCIATION CELLULAIRE

(30) Priority: 19.01.2017 JP 2017007366
(43) Date of publication of application: 27.11.2019
(73) Proprietor: Sysmex Corporation, Kobe-shi, Hyogo 651-0073 (JP)
(72) Inventor: AIHARA, Yuki, Kobe-shi Hyogo 651-0073 (JP)
(74) Representative: De Clercq & Partners
(86) International application number: PCT/JP2017/047160
(87) International publication number: WO 2018/135288

(56) References cited:
- WO-A1-2015/052527
- WO-A1-2016/010119
- JP-A- 2012 508 577
- JP-A- 2013 535 979
- JP-A- 2015 500 013
- JP-A- 2015 529 450
- JP-A- 2015 534 830
- US-A1- 2012 178 085
- ROSIE SARAH GRIFFITHS: "MicroRNA Regulation of Chondrogenesis in Human Embryonic Stem Cells", 2016, XP055736928, Retrieved from the Internet <URL:https://www.escholar.manchester.ac.uk/api/datastream?publicationPid=uk-ac-man-scw:306915&datastreamId=FULL-TEXT.PDF> [retrieved on 20201006]
- MI-RA SUH ET AL: "Human embryonic stem cells express a unique set of microRNAs", DEVELOPMENTAL BIOLOGY, vol. 270, no. 2, 1 June 2004 (2004-06-01), AMSTERDAM, NL, pages 488 - 498, XP055546408, ISSN: 0012-1606, DOI: 10.1016/j.ydbio.2004.02.019
- YING ZHANG ET AL: "A non-invasive method to determine the pluripotent status of stem cells by culture medium microRNA expression detection", SCIENTIFIC REPORTS, vol. 6, no. 1, 1 April 2016 (2016-04-01), XP055627860, DOI: 10.1038/srep22380
- NAKAMURA YOSHIHIRO ET AL: "Mesenchymal-stem-cell-derived exosomes accelerate skeletal muscle regeneration", FEBS LETTERS, ELSEVIER, AMSTERDAM, NL, vol. 589, no. 11, 8 April 2015 (2015-04-08), pages 1257 - 1265, XP029219936, ISSN: 0014-5793, DOI: 10.1016/J.FEBSLET.2015.03.031
- ZHONGHUI ZHANG ET AL: "MicroRNA-302/367 Cluster Governs hESC Self-Renewal by Dually Regulating Cell Cycle and Apoptosis Pathways", STEM CELL REPORTS, vol. 4, no. 4, 1 April 2015 (2015-04-01), United States, pages 645 - 657, XP055737231, ISSN: 2213-6711, DOI: 10.1016/j.stemcr.2015.02.009

## Description

### TECHNICAL FIELD

The present invention relates to a method for evaluating the differentiation state of cells.

### BACKGROUND ART

In regenerative medicine, differentiation of pluripotent stem cells into desired cells is performed. However, even if a differentiation-inducing operation is performed, not all the pluripotent stem cells are differentiated. Some pluripotent stem cells may remain undifferentiated. If cell transplantation is performed with such residual pluripotent stem cells, a tumor may occur from the transplanted cells. Therefore, it is necessary to evaluate the differentiation state of cells in a culture medium.

A method disclosed in Non-Patent Literature 1 has been known as a technique of detecting hiPSCs (human induced pluripotent stem cells) after a differentiation-inducing operation. In the method disclosed in Non-Patent Literature 1, after the differentiation-inducing operation, some cells are collected and subjected to RNA extraction, and an expression level of LIN28 mRNA is measured through qRT-PCR. Based on the result of the measurement, residual hiPSCs are detected. However, since all the cells prepared for regenerative medicine are valuable, destructive inspection in which some cells are consumed for evaluation of quality of differentiated cells, as described in Non-Patent Literature 1, is not preferable. Non-Patent Literature 2 discloses a method as an example of nondestructive inspection. Non-Patent Literature 2 discloses detection of residual pluripotent stem cells by using, as an index, hyperglycosylated podocalyxin that is present in a culture supernatant after a differentiation-inducing operation.

Ying Zhang et al teaches a non-invasive method to determine the pluripotent status of stem cells by culture medium microRNA expression detection (SCIENTIFIC REPORTS, vol. 6, Article number: 22380, 1 April 2016).

Mi-Ra Suh et al teaches that human embryonic stem cells express a unique set of microRNAs, including miRNAs from the miR302/367 cluster (DEVELOPMENTAL BIOLOGY, vol. 270, no. 2, 1 June 2004, pages 488-498).

### CITATION LIST

### [NON PATENT LITERATURE]

[NPL 1] Kuroda, et al., PLoS ONE, 2012, May, Volume 7(5), e37342
[NPL 2] Tateno, et al., SCIENTIFIC REPORTS, 2014, 4, 4069

### SUMMARY OF THE INVENTION

### PROBLEMS TO BE SOLVED BY THE INVENTION

An object of the present invention is to provide a method capable of detecting pluripotent stem cells with enhanced sensitivity without destructing cells.

### SOLUTION TO THE PROBLEMS

The present invention provides a method for evaluating a differentiation state of cells, including: measuring at least one miRNA in a miR302/367 cluster in a liquid phase fraction of a cell culture solution during differentiation induction of pluripotent stem cells and/or after the differentiation induction; and evaluating a differentiation state of the cells in the cell culture solution on the basis of a miRNA measurement value , wherein the liquid phase fraction of a cell culture solution is obtained by separation of the cells from the cell culture solution.

. The inventors of the present invention have newly found that pluripotent stem cells release miRNAs in a miR302/367 cluster outside the cells, and that the differentiation state of the cells can be evaluated by measuring miRNAs in the miR302/367 cluster in a liquid phase fraction of a cell culture solution.

Disclosed but not part of the invention is a method for evaluating a differentiation state from pluripotent stem cells into differentiated cells. The method includes obtaining: a measurement value of miRNAs in a miR302/367 cluster in a liquid phase fraction of a cell culture solution that has been known to contain pluripotent stem cells; a measurement value of miRNAs in a miR302/367 cluster in a liquid phase fraction of a cell culture solution that has been known to contain no pluripotent stem cells and contain differentiated cells, and a measurement value of miRNAs in a miR302/367 cluster in a liquid phase fraction of a cell culture solution that contains cells the differentiation state of which is unknown. The method further includes comparing these measurement values to evaluate the differentiation state of the cells of which the differentiation state is unknown.

The present invention provides a method for detecting pluripotent stem cells. The method includes the steps of: measuring at least one miRNA in a miR302/367 cluster in a liquid phase fraction of a cell culture solution during differentiation induction of pluripotent stem cells and/or after the differentiation induction; and detecting pluripotent stem cells in the cell culture solution on the basis of a miRNA measurement value , wherein the liquid phase fraction of a cell culture solution is obtained by separation of the cells from the cell culture solution. .

Disclosed but not part of the invention is a method for measuring miRNAs. The method includes the steps of: inducing differentiation of pluripotent stem cells in a cell culture solution; and obtaining a measurement value of miRNAs in a miR302/367 cluster in a liquid phase fraction of a cell culture solution during the differentiation induction and/or after the differentiation induction.

### ADVANTAGEOUS EFFECTS OF THE INVENTION

According to the present invention, a method capable of detecting pluripotent stem cells with enhanced sensitivity without substantially destroying cells is provided, as defined in the claims.

### BRIEF DESCRIPTION OF THE DRAWINGS

[FIG. 1] FIG. 1 is a graph showing a result of Example 1.
[FIG. 2] FIG. 2 is a graph showing a measurement result of miR302a in Example 2.
[FIG. 3] FIG. 3 is a graph showing a measurement result of miR302b in Example 2.
[FIG. 4] FIG. 4 is a graph showing a measurement result of miR302c in Example 2.
[FIG. 5] FIG. 5 is a graph showing a measurement result of miR302d in Example 2.
[FIG. 6] FIG. 6 is a graph showing a measurement result of miR367 in Example 2.
[FIG. 7] FIG. 7 is a graph showing a measurement result of miR371 in Comparative Example 1.

### BEST MODE FOR CARRYING OUT THE INVENTION

A sample used in a method according to an embodiment of the present invention is a liquid phase fraction of a cell culture solution that is used when differentiation of pluripotent stem cells is induced. This liquid phase fraction is a liquid phase fraction of a cell culture solution in the middle of the differentiation-inducing operation and/or after the differentiation-inducing operation. In this specification, "cell culture solution" is a liquid culture medium for cell culture and differentiation, contained in an incubator. The cell culture solution includes a culture medium, and pluripotent stem cells and/or differentiated cells. "Liquid phase fraction" is the whole or part of a solution portion of a cell culture solution during differentiation induction and/or after differentiation induction, and does not substantially include cells.

Pluripotent stem cells are stem cells that have a capability (pluripotency) of differentiating into cells derived from ectoderm, endoderm, and mesoderm. The pluripotent stem cells also have proliferation potency. Examples of the pluripotent stem cells include: induced pluripotent stem cell (iPS cell); embryonic stem cell (ES cell); non-human embryonic stem cell (ntES cell) derived from a cloned non-human embryo obtained through nuclear transfer; and embryonic germ cell (EG cell).

iPS cell is a stem cell that is created by introducing a specific reprogramming factor (DNA or protein) into a somatic cell such as a skin cell. iPS cell has pluripotency and proliferation potency. ES cell is a stem cell that can be derived from an inner cell mass of a blastocyst of a mammal. ES cell has pluripotency and proliferation potency. ntES cell is a ES cell established from an inner cell mass of a blastocyst that is derived from a cloned embryo obtained by substituting the nucleus of an unfertilized egg with the nucleus from a somatic cell. ntES cell has substantially the same characteristics as ES cell. Human ntES cells and their uses are excluded and do not fall within the scope of the present invention. EG cell is a cell, having pluripotency similar to that cell, which is established from a primordial germ cell at the prenatal period. EG cell can be established by culturing a primordial germ cell in the presence of a substance such as LIF, bEGF, a stem cell factor, or the like.

Pluripotent stem cells may be pluripotent stem cells collected from a living organism, with the exception of the human embryo, or may be cells that are artificially prepared by reprogramming differentiated cells collected from a living organism. From the viewpoint of regenerative medicine research and clinical application, pluripotent stem cells are preferably artificially prepared cells. The origins of pluripotent stem cells are not particularly limited. However, pluripotent stem cells are preferably derived from mammals such as human, monkey, dog, cat, horse, mouse, rat, hamster, guinea pig, rabbit, sheep, pig, and cow.

Differentiation induction is an operation of culturing pluripotent stem cells in a culture medium containing a factor that induces differentiation of stem cells, and differentiating the cells. As a cell culture solution, any of known culture media can be used. Examples of culture media include IMDM, Medium 199, EMEM, αMEM, DMEM, Ham's F12, RPMI 1640, Fischer's culture medium, MEF-CM, StemPro^{™} 34 (Invitrogen), Essential 8^{™} (Thermo Fisher Scientific), hPSC Growth Medium DXF^{™} (Takara Bio Inc.), StemFit AK02N^{™} (Takara Bio Inc.), and a culture medium obtained by mixing any of the above media.

As a differentiation induction factor, a known substance can be used depending on the type of differentiated cells. The cell culture solution may contain components required for proliferation/maintenance of cells, in addition to the differentiation induction factor. Examples of the components include serum, albumin, transferrin, Knockout Serum Replacement (KSR CO. LTD.), N2 supplement (Invitrogen), B27 supplement (Invitrogen), fatty acid, insulin, collagen precursor, 2-mercapto-ethanol, thiol glycerol, lipid, amino acid, L-glutamine, Glutamax^{™} (Invitrogen), non-essential amino acid, vitamin, proliferation factor, antibiotic, antioxidant, pyruvic acid, buffer agent, mineral salt, ROCK inhibitor, and Wnt signal inhibitor.

The method of differentiation induction is not particularly limited. Usually, suspension culture, adhesion culture, or a combination thereof is used. The suspension culture is a type of culture that is performed under a condition that cells are non-adhesive to an incubator. In the suspension culture, cells are not necessarily dispersing in the cell culture solution and may sediment on a bottom part of the incubator. The incubator used for the suspension culture is not particularly limited. Examples of the incubator include a flask, dish, plate, chamber, and tube. The incubator used for the suspension culture is preferably non-cell-adhesive. The incubator is preferably formed of a hydrophobic material, or coated with a coating agent (e.g., polyhydroxyethyl methacrylate copolymer) that prevents adhesion of cells to the surface thereof.

The adhesion culture is a type of culture in which pluripotent stem cells are adhered to a support body. An incubator is used as the support body. The incubator used for the adhesion culture is not particularly limited. For the adhesion culture, the same incubator as that used for the suspension culture can be used. However, the surface of the incubator is preferably coated with a coating agent (also referred to as "cell culture substrate", "adhesion substrate", or the like) that causes cells to adhere to the surface of the incubator. Examples of the coating agent that causes the cells to adhere to the surface of the incubator include gelatin, laminin, collagen, Poly-D-Lysine, polyornithine, fibronectin, and vitronectine. A commercial product such as iMatrix-511 (Nippi Inc.) containing Laminin-511 E8 fragment can also be used. A known scaffold can also be used as the support body. In this case, pluripotent stem cells proliferate with the scaffold as a stage, thereby realizing three-dimensional culture. A commercial product can be used as the scaffold. Examples of the scaffold include: Matrigel^{™} (Corning); QGel^{™} MT 3D Matrix (Qgel SA); 3-D Life Biomimetic (Cellendes); Puramatrix (3D MATRIX); and alvetex (Reinnervate).

The culture condition can be adjusted as appropriate depending on the type of the pluripotent stem cells and the type of the differentiated cells. For example, a preferable culture temperature is about 30 to 40°C, and a preferable carbon dioxide concentration is about 1 to 10%.

In the method according to the present embodiment, as a sample for miRNA measurement, a liquid phase fraction of a cell culture solution is collected. The method for extracting a liquid phase fraction from a cell culture solution is not particularly limited.

When differentiation induction is performed by suspension culture, a cell culture solution containing cells is centrifuged, and a supernatant of the centrifuged solution can be obtained as a liquid phase fraction of the present embodiment. Since the cells sediment in the cell culture solution, the liquid phase fraction may be obtained from a lower part of the incubator while dispersing the cells. The cell culture solution containing the cells may be filtered and separated into a fraction containing the cells and a liquid phase fraction to obtain the liquid phase fraction. If cells are likely to be mixed in the obtained liquid phase fraction, the cells may be separated/removed through further centrifugation or filtering.

When differentiation induction is performed by adhesion culture, since the cells are adhered to the bottom part of the incubator, a liquid phase fraction of the incubator can be sucked by using a pipet or the like. If cells separated from the bottom part are likely to be present in the liquid phase fraction, the liquid phase fraction may be centrifuged to separate/remove the cells.

The cells obtained through the differentiation-inducing operation are not particularly limited and may be any cells as long as they have lost pluripotency through differentiation of pluripotent stem cells. In this specification, cells obtained by differentiating pluripotent stem cells are referred to as "differentiated cells". Examples of the differentiated cells include: cells having multipotency such as hematopoietic stem cells; cells having oligopotency such as neural stem cells; cells having unipotency such as progenitor cells; and terminally differentiated cells. In this specification, cells having no differentiation property are referred to as "terminally differentiated cells". Examples of the terminally differentiated cells include cells from skin, retina, cornea, inner/outer ear, brain, spinal cord, peripheral nerve, adrenal medulla (cell derived from ectoderm), corium, skeletal muscle, bone, cartilage, kidney, blood cell, vascular endothelial, adrenal cortex (cell derived from mesoderm), stomach, liver, pancreas, bowel, and thyroid gland (cell derived from endoderm).

miRNAs, which are measurement targets in the present embodiment, are miRNAs in a miR302/miR367 cluster. A gene group that codes human miR302 and miR367 is located in an intron of LARP7 gene on a genome to form a miR302/miR367 cluster. In this specification, "miRNAs in a miR302/367 cluster" mean miRNAs transcribed from these genes. The miR302 forms a family including miR302a, miR302b, miR302c, and miR302d, and is also called a miR302 family. After being transcribed from the genes, each miRNA is matured through pri-miRNA and pre-miRNA. In the present embodiment, preferably, the mature miRNA is measured. Nucleotide sequences of mature miRNAs of the miR302a, the miR302b, the miR302c, and the miR302d are represented by SEQ ID NOs. 1 to 4. A nucleotide sequence of mature miRNA of the miR367 is represented by SEQ ID NO. 5. The genes that code these miRNAs are disclosed as shown on Table 1 in the GenBank database.

**[Table 1]**

| Gene | GenBank Accession No. |
|---|---|
| miR302a gene | NR_029835.1 |
| miR302b gene | NR_029857.1 |
| miR302c gene | NR_029858.1 |
| miR302d gene | NR_029859.1 |
| miR367 gene | NR_029860.1 |

The miRNA measurement method is not particularly limited, and a known method can be used. Examples of the measurement method include: a measurement method using hybridization of a probe; a measurement method using a nucleic acid amplification technique; a measurement method using mass spectrometry; and a measurement method using sequencing. Examples of the measurement method using hybridization of a probe include: a microarray method; northern hybridization; and RNase protection assay. Examples of the measurement method using the nucleic acid amplification technique include: quantitative RT-PCR and quantitative RT-LAMP. Examples of the quantitative RT-PCR include SYBR^{™} Green and TaqMan^{™}. Since a miRNA to be amplified is short, when nucleic acid amplification is used, a stem-loop primer or poly (A) addition is usually adopted.

In the present embodiment, pluripotent stem cells are detected based on a measurement value of miRNAs in a miR302/miR367 cluster. Since the miRNAs in the miR302/miR367 cluster bind to specific mRNAs in the pluripotent stem cells and exhibit a function of inhibiting translation thereof to protein, it has been known that such miRNAs are present in the cells. However, it has not been known that the miRNAs are released to the outside of the pluripotent stem cells. Since miRNAs in the miR302/miR367 cluster are not expressed in differentiated cells, it is possible to detect pluripotent stem cells on the basis of the measurement value of the miRNAs in the miR302/miR367 cluster.

In this specification, "measurement value" means a value that reflects the amount of miRNAs existing in a liquid phase fraction. Examples of the measurement value include: an optical measurement value (fluorescence intensity, turbidity, absorbance, etc.); a reaction cycle number or reaction time when the optical measurement value reaches a predetermined reference value; and a quantitative value (copy number, mass, or concentration) of miRNAs calculated using a calibration curve. The predetermined reference value can be set as appropriate, depending on the type of a value representing the amount of the miRNAs or the concentration of the miRNAs. For example, when quantitative RT-PCR is used, an amount of change in the fluorescence intensity when a nucleic acid amplification reaction indicates logarithmic amplification can be set with a predetermined cycle number being a reference value.

The differentiation-inducing operation usually takes several hours to several months to complete. In one embodiment, a liquid phase fraction of a cell culture solution obtained after start of differentiation induction and before completion of the differentiation induction, i.e., obtained during the differentiation induction, is subjected to miRNA measurement. In this case, by detecting residual pluripotent stem cells, the state of differentiation of the pluripotent stem cells during the differentiation-inducing operation can be monitored. For example, miRNAs in a miR302/miR367 cluster are measured before start of the differentiation induction and during the differentiation induction, and the measurement values are compared to evaluate the progress state of differentiation. More specifically, if there is no great change between the measurement value of miRNAs in the miR302/miR367 cluster before start of the differentiation induction and that during the differentiation induction, it is determined that the differentiation induction is not appropriately performed, and the differentiation operation can be suspended. If miRNAs in the miR302/miR367 cluster become no longer detected during the differentiation induction, it is determined that all the pluripotent stem cells have been differentiated and the differentiation-inducing operation can be completed.

In another embodiment, at a plurality of times during differentiation induction, miRNAs in a miR302/miR367 cluster are measured, and the measurement values are compared to monitor the progress state of differentiation. Specifically, at a first time point during the differentiation induction, a liquid phase fraction is collected from a cell culture solution, and miRNAs in the miR302/miR367 cluster are measured to obtain a first measurement value. At a second time point during the differentiation induction, a liquid phase fraction is collected from the cell culture solution, and miRNAs in the miR302/miR367 cluster are measured to obtain a second measurement value. The first measurement value and the second measurement value are compared to evaluate the differentiation state.

In still another embodiment, a liquid phase fraction of a cell culture solution after completion of the differentiation-inducing operation is subjected to miRNA measurement. Even if the differentiation-inducing operation itself is completed, not all the pluripotent stem cells are differentiated, and a few pluripotent stem cells may remain undifferentiated. Since such residual pluripotent stem cells have the risk of generating a tumor as described above, the residual pluripotent stem cells are detected through quality inspection for cell aggregation after completion of the differentiation-inducing operation. The detection result can be used for determination of tumorigenic risk, for example. The determination of tumorigenic risk is performed as follows. For example, miRNAs in the miR302/miR367 cluster are detected from the liquid phase fraction after the differentiation induction. When it is determined that pluripotent stem cells remain undifferentiated, the tumorigenic risk of the cells after the differentiation induction is determined to be high. In this case, transplant is suspended, and it can be determined to continue the differentiation induction, for example. When it is determined that no pluripotent stem cells remain undifferentiated, the tumorigenic risk of the cells after the differentiation induction is determined to be low. In this case, it can be determined to transplant the cells after the differentiation induction into an organism, for example.

In yet another embodiment, detection of pluripotent stem cells can be performed during differentiation induction and after the differentiation induction. In this case, monitoring of the differentiation state during the differentiation induction and quality inspection for the cells after the differentiation induction, can be performed.

In any of the above embodiments, when the culture medium is replaced with a new culture medium when the differentiation induction is executed, the old culture medium may be subjected to miRNA measurement.

In this specification, "detecting" includes: qualitatively determining presence/absence of pluripotent stem cells; quantitatively determining pluripotent stem cells; and semi-quantitatively detecting the amount of existing pluripotent stem cells. The semiquantitative detection means indicating the amount of existing pluripotent stem cells in a stepwise manner, such as "-", "+", "++" (negative, weakly positive, strongly positive).

In determining presence/absence of pluripotent stem cells, for example, the measurement value of the miRNAs in the miR302/miR367 cluster in the liquid phase fraction can be compared with a predetermined threshold. When the measurement value of the miRNAs in the miR302/miR367 cluster is not less than the threshold, it is determined that pluripotent stem cells are present. When the measurement value is less than the threshold, it is determined that no pluripotent stem cells are present.

When quantitatively determining pluripotent stem cells, for example, the number of pluripotent stem cells can be calculated based on a calibration curve from the measurement value of the miRNAs in the miR302/miR367 cluster in the liquid phase fraction.

When semi-quantitatively detecting pluripotent stem cells, determination can be made by comparing the measurement value with a plurality of thresholds. For example, when the measurement value is less than a first threshold, it is determined that no pluripotent stem cells are present ("-"). When the measurement value is not less than the first threshold and less than the second threshold, it is determined that a small amount of pluripotent stem cells are present ("+"). When the measurement value is not less than the second threshold, it is determined that the amount of existing pluripotent stem cells is "++".

The thresholds used in the present embodiment are preferably set in advance of executing the method of the present embodiment. For example, a plurality of liquid phase fractions of a cell culture solution containing pluripotent stem cells and a plurality of liquid phase fractions of a cell culture solution containing no pluripotent stem cells are prepared, and miRNAs in a miR302/miR367 cluster are measured for each liquid phase fraction, whereby a threshold can be set to a value that allows determination of presence/absence of pluripotent stem cells to be performed at the highest accuracy.

When executing the method of the present embodiment, a liquid phase fraction (positive control) of a cell culture solution that has been known to contain pluripotent stem cells, a liquid phase fraction (negative control) of a cell culture solution that has been known to contain no pluripotent stem cells, and a liquid phase fraction (unknown sample) of a cell culture solution in which presence/absence of pluripotent stem cells is unknown, may be prepared, and miRNAs in a miR302/miR367 cluster in each liquid phase fraction may be measured. If the miRNA measurement value in the unknown sample is closer to the miRNA measurement value in the positive control than to the miRNA measurement value in the negative control, it can be determined that the unknown sample is prepared from the cell culture solution in which pluripotent stem cells are present. If the miRNA measurement value in the unknown sample is closer to the miRNA measurement value in the negative control than to the miRNA measurement value in the positive control, it can be determined that the unknown sample is prepared from the cell culture solution in which no pluripotent stem cells are present.

Another embodiment relates to a reagent or a reagent kit that contains an oligonucleotide probe and/or an oligonucleotide primer which can detect miRNAs in a miR302/miR367 cluster. These oligonucleotides may bind to a solid phase such as a plate or particles. When measuring a mature miRNA by using the oligonucleotide primer, since the mature miRNA has a short nucleotide length, a stem loop primer can be used as a forward primer and/or a reverse primer. The reagent kit may contain another reagent such as a buffer solution.

Still another embodiment relates to use of an oligonucleotide probe or an oligonucleotide primer for manufacturing a reagent or a reagent kit for miRNA measurement described above.

The present disclosure includes a method of measuring miRNAs in a miR302/367 cluster. In one embodiment of this method, differentiation of pluripotent stem cells is induced in a cell culture solution. miRNAs in a miR302/367 cluster contained in a liquid phase fraction of the cell culture solution during the differentiation induction and/or after the differentiation induction are measured.

The present invention will be described in detail below with reference to Examples, but the present invention is not limited to the Examples. The invention is as defined in the appended claims.

Example 1: Comparison in miR302 measurement value between a culture supernatant of iPS cells and a culture supernatant of cancer cells

### (1) Preparation of measurement sample

15 mL of Essential 8 (manufactured by Gibco) as a cell culture solution was added into a 75 cm² flask (manufactured by Corning) the inside of which was coated with iMatrix-511 (Nippi Inc.) as a cell culture substrate. iPS cells (10⁵ cells/1.5 mL) were added to the cell culture solution, and subjected to adhesion culture for a day. After the culture, a supernatant was collected.

Likewise, a colon cancer cell line, HCT116 (10⁵ cells /1.5 mL), was subjected to adhesion culture for a day. The culture solution was centrifuged at 1500 g for 10 minutes, and a supernatant was collected.

50 µL of the collected supernatant and 50 µL of negative control (Essential 8 that contained no cells and was not subjected to cell culture) were used as measurement samples.

### (2) miR302 measurement

Total RNA was collected from each measurement sample by using High Pure miRNA isolation kit (manufactured by Roche Diagnostics). Quantitative RT-PCR was performed by using 1 µL of the total RNA to measure each of miR302a, miR302b, miR302c, and miR302d. The quantitative RT-PCR was performed by using TaqMan^{™} MicroRNA Reverse Transcription Kit, TaqMan^{™} MicroRNA Assays, TaqMan^{™} Universal Master Mix II, and Applied Biosystems^{™} 7500 fast (manufactured by Thermo Fisher Scientific), in accordance with manufacturer's instructions.

### (3) Results

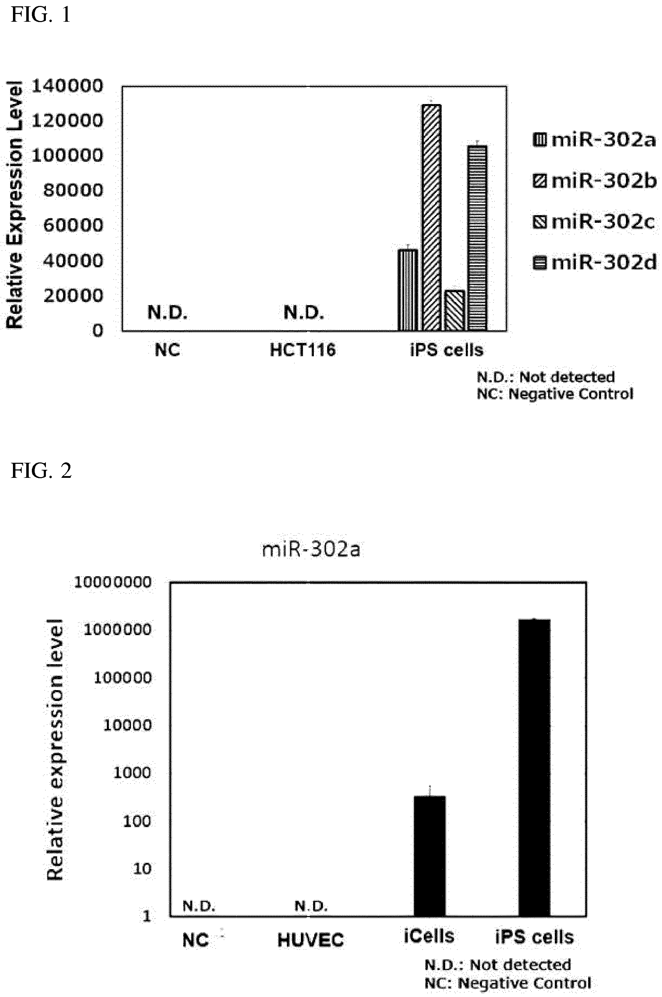

The results are shown in FIG. 1. The vertical axis of the graph shown in FIG. 1 indicates the relative expression level of miR302 when a Ct value of 40 is expression level 1. As is understood from FIG. 1, no miR302 was detected from the negative control and the culture supernatant of colon cancer cells, while miR302a, miR302b, miR302c, and miR302d were detected from the culture supernatant of iPS cells.

Example 2: Comparison in miRNA measurement value in a miR302/367 cluster among a culture supernatant of iPS cells, a culture supernatant of vascular endothelial cells (derived from iPS cells), and a culture supernatant of vascular endothelial cells (cell lines)

### (1) Preparation of measurement sample

A culture supernatant of iPS cells was obtained in the same manner as described in Example 1. 15mL of VascuLife VEGF Medium Complete Kit (manufactured by KURABO INDUSTRIES LTD.) as a cell culture solution was added into a 75 cm² flask (manufactured by Corning) the inside of which was coated with fibronectin (manufactured by Invitrogen) as a cell culture substrate. iCell^{™} Endothelial Cells (manufactured by Cellular Dynamics International) (10⁵ cells/1.5 mL), which were vascular endothelial cells differentiated from iPS cells, were added to the cell culture solution, and subjected to adhesion culture for a day. After the culture, the culture solution was centrifuged at 1500g for 10 minutes, and a supernatant was collected.

HUVEC (10⁵ cells/1.5 mL) as a vascular endothelial cell line was similarly cultured, and the culture solution was centrifuged to collect a supernatant.

50 µL of the collected supernatant and 50 µL of negative control (VascuLife VEGF Medium that contained no cells and was not subjected to cell culture) were used as measurement samples.

### (2) miRNA measurement

Using the measurement samples prepared in Example 2 (1), miRNA measurement for miR302 and miR367 was in the same manner as that of Example 1.

### (3) Results

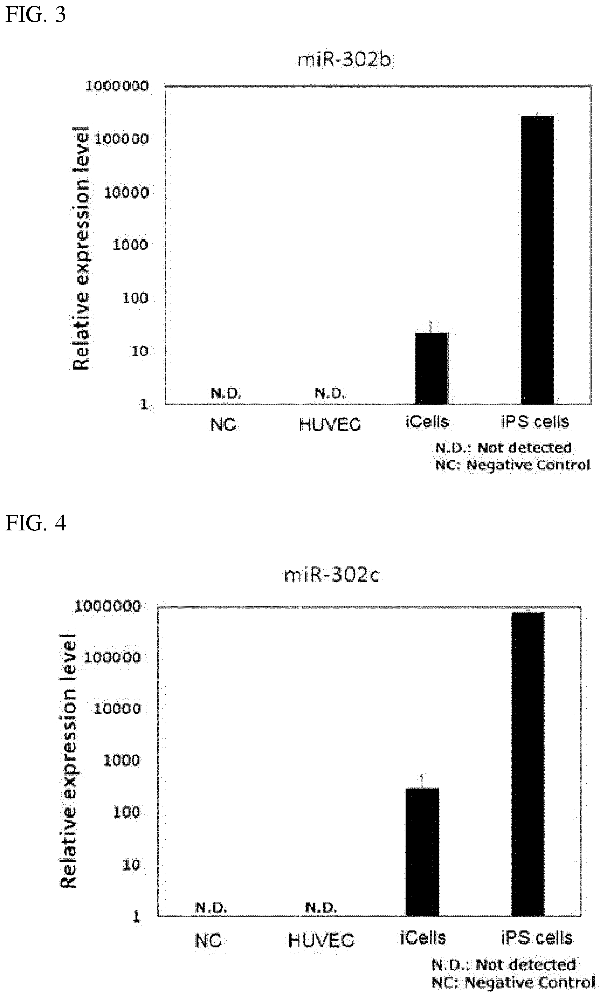

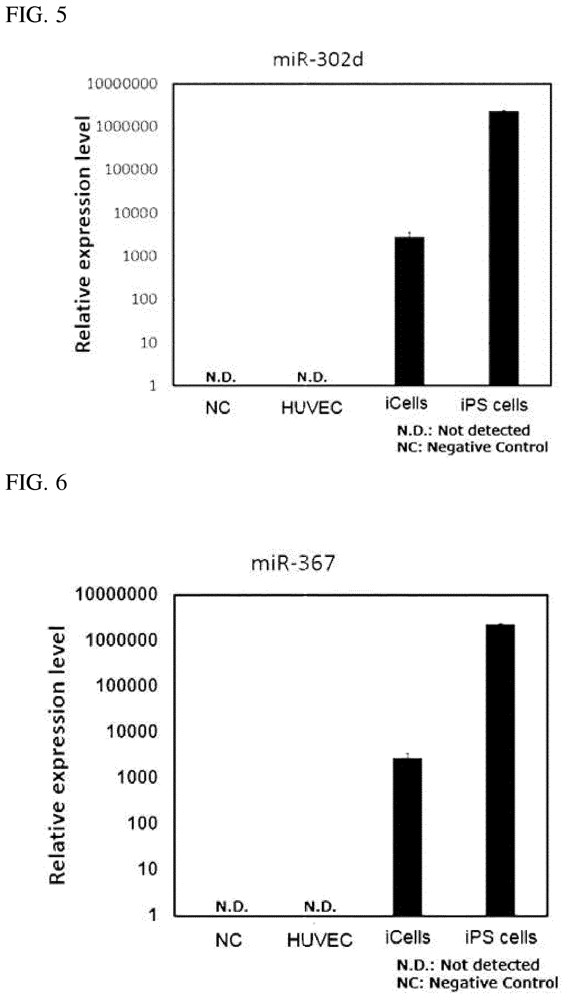

The results are shown in FIGS. 2 to 6. FIGS. 2 to 6 are graphs showing the miRNA measurement values of miR302a to miR302d and miR367, respectively. The vertical axis of each graph indicates the relative expression level of miRNA when a Ct value of 45 is an expression level 1. As is understood from FIGS. 2 to 6, no miRNA was detected from the negative control. The vascular endothelial cells, HUVEC, are cell lines, and contain no pluripotent stem cells. No miRNA was detected from the culture supernatant of HUVEC. From the culture supernatant of iPS cells, all of miR302a, miR302b, miR302c, miR302d, and miR367 were detected and showed high measurement values. From the culture supernatant of iCell Endothelial Cells obtained through differentiation induction from iPS cells, an expression level lower than that of the culture supernatant of iPS cells was detected. The package insert of iCell Endothelial Cells describes that iCell Endothelial Cells have a purity of 98% (expression rate of vascular endothelial cell marker). According to the above results, it was suggested that iPS cells, which remained undifferentiated, could be detected after completion of differentiation-inducing operation into vascular endothelial cells.

Comparative Example 1: Comparison in measurement values of miR371, miR372, and miR373 among a culture supernatant of iPS cells, a culture supernatant of vascular endothelial cells (derived from iPS cells), and a culture supernatant of vascular endothelial cells (cell lines)

In Comparative Example 1, the expression levels of miR371, miR372, and miR373, which were known as pluripotent stem cell markers, were compared. Nucleotide sequences of mature miRNAs of miR371, miR372, and miR373 are represented by SEQ ID NOs. 6 to 8.

### (1) Preparation of measurement samples

Measurement samples and negative controls were prepared in the same manner as described in Example 2.

### (2) miRNA measurement

Using the measurement samples prepared in Example 3 (1), miRNA measurement for miR371, miR372, and miR373 was performed in the same manner as described in Example 2.

### (3) Results

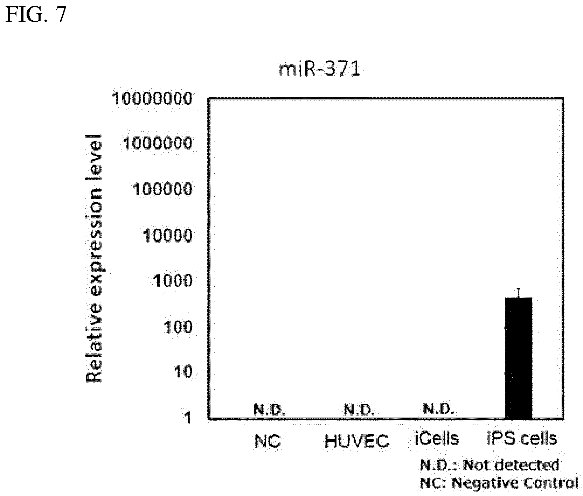

FIG. 7 shows the measurement results for miR371. FIG. 7 is a graph showing the miRNA measurement value of miR371. The vertical axis of the graph indicates the relative expression level of miRNA when a Ct value of 45 is expression level 1. As is understood from FIG. 1, no miRNA was detected from the culture supernatant of iCell Endothelial Cells obtained through differentiation induction from iPS cells, while a small amount of miRNAs were detected from the culture supernatant of iPS cells. Neither miR372 nor miR373 was detected from any of the culture supernatants.

From the results of Examples and Comparative Example, it is understood that, among molecules known as pluripotent stem cell markers, some molecules are released from cells while the others are not released from cells. In Examples described above, miRNAs in a miR302/367 cluster were found as molecules that are releasable from cells, and it was verified that the miRNAs were usable as markers representing the differentiation states of cells.

## Claims

1. A method for evaluating a differentiation state of cells, comprising:
measuring at least one miRNAin a miR302/367 cluster in a liquid phase fraction of a cell culture solution during differentiation induction of pluripotent stem cells and/or after the differentiation induction; and
evaluating a differentiation state of the cells in the cell culture solution on the basis of a miRNA measurement value,
wherein the liquid phase fraction of a cell culture solution is obtained by separation of the cells from the cell culture solution.

2. The method according to claim 1, wherein the pluripotent stem cells are iPS cells, ES cells, non-human ntES cells, or EG cells.

3. The method according to any one of claims 1 or 2, wherein the miRNA measurement value is a miR302a measurement value, a miR302b measurement value, a miR302c measurement value, a miR302d measurement value, a miR367 measurement value, or a sum of at least two of the measurement values.

4. The method according to any one of claims 1 to 3, wherein the measurement value is obtained through quantitative RT-PCR.

5. The method according to any one of claims 1 to 4, wherein
the miRNA measurement value is compared with a predetermined threshold,
when the miRNA measurement value is not smaller than the threshold, it is determined that pluripotent stem cells are contained in the cell culture solution, and
when the miRNA measurement value is smaller than the threshold, it is determined that no pluripotent stem cells are contained in the cell culture solution.

6. The method according to any one of claims 1 to 5, wherein the number of the pluripotent stem cells in the cell culture solution is calculated based on the miRNA measurement value.

7. The method according to any one of claims 1 to 6, wherein the miRNA measurement comprises: miRNA measurement at a first time point during the differentiation induction of the pluripotent stem cells; and miRNA measurement at a second time point during the differentiation induction of the pluripotent stem cells.

8. A method for detecting pluripotent stem cells, comprising the steps of:
measuring at least one miRNA in a miR302/367 cluster in a liquid phase fraction of a cell culture solution during differentiation induction of pluripotent stem cells and/or after the differentiation induction; and
detecting pluripotent stem cells in the cell culture solution on the basis of a miRNA measurement value,
wherein the liquid phase fraction of a cell culture solution is obtained by separation of the cells from the cell culture solution.

9. The method according to any one of claims 1 to 8, wherein the pluripotent stem cells are cells that have been prepared by reprogramming differentiated cells collected from an organism.

10. The method according to any one of claims 1 to 9, wherein the miRNAs are RNAs described in SEQ ID NOs. of 1 to 5.

## Patentansprüche

1. Verfahren zur Bewertung eines Differenzierungszustands von Zellen, umfassend:
Messen mindestens einer miRNA in einem miR302/367-Cluster in einer Flüssigphasenfraktion einer Zellkulturlösung während der Differenzierungsinduktion pluripotenter Stammzellen und/oder nach der Differenzierungsinduktion; und
Bewerten eines Differenzierungszustands der Zellen in der Zellkulturlösung anhand eines miRNA-Messwerts,
wobei die Flüssigphasenfraktion einer Zellkulturlösung durch Trennung der Zellen von der Zellkulturlösung erhalten wird.

2. Verfahren nach Anspruch 1, wobei es sich bei den pluripotenten Stammzellen um iPS-Zellen, ES-Zellen, nichtmenschliche ntES-Zellen oder EG-Zellen handelt.

3. Verfahren nach Anspruch 1 oder Anspruch 2, wobei es sich bei dem miRNA-Messwert um einen miR302a-Messwert, einen miR302b-Messwert, einen miR302c-Messwert, einen miR302d-Messwert, einen miR367-Messwert oder eine Summe aus mindestens zwei der Messwerte handelt.

4. Verfahren nach einem der Ansprüche 1 bis 3, wobei der Messwert über quantitative RT-PCR erhalten wird.

5. Verfahren nach einem der Ansprüche 1 bis 4, wobei der miRNA-Messwert mit einem vorbestimmten Schwellenwert verglichen wird,
wenn der miRNA-Messwert nicht kleiner als der Schwellenwert ist, bestimmt wird, dass pluripotente Stammzellen in der Zellkulturlösung enthalten sind, und, wenn der miRNA-Messwert kleiner als der Schwellenwert ist, bestimmt wird, dass keine pluripotenten Stammzellen in der Zellkulturlösung enthalten sind.

6. Verfahren nach einem der Ansprüche 1 bis 5, wobei die Anzahl der pluripotenten Stammzellen in der Zellkulturlösung auf Grundlage des miRNA-Messwerts berechnet wird.

7. Verfahren nach einem der Ansprüche 1 bis 6, wobei die miRNA-Messung Folgendes umfasst: miRNA-Messung zu einem ersten Zeitpunkt während der Differenzierungsinduktion der pluripotenten Stammzellen; und miRNA-Messung zu einem zweiten Zeitpunkt während der Differenzierungsinduktion der pluripotenten Stammzellen.

8. Verfahren zum Nachweisen pluripotenter Stammzellen, umfassend die Schritte:
Messen mindestens einer miRNA in einem miR302/367-Cluster in einer Flüssigphasenfraktion einer Zellkulturlösung während der Differenzierungsinduktion pluripotenter Stammzellen und/oder nach der Differenzierungsinduktion; und
Nachweisen pluripotenter Stammzellen in der Zellkulturlösung anhand eines miRNA-Messwerts,
wobei die Flüssigphasenfraktion einer Zellkulturlösung durch Trennung der Zellen von der Zellkulturlösung erhalten wird.

9. Verfahren nach einem der Ansprüche 1 bis 8, wobei es sich bei den pluripotenten Stammzellen um Zellen handelt, die durch Umprogrammieren von einem Organismus entnommenen differenzierten Zellen hergestellt wurden.

10. Verfahren nach einem der Ansprüche 1 bis 9, wobei es sich bei den miRNAs um RNAs gemäß SEQ ID NO. 1 bis 5 handelt.

## Revendications

1. Procédé d'évaluation d'un état de différenciation de cellules comprenant :
la mesure d'au moins un miARN dans un cluster miR302/367 dans une fraction en phase liquide d'une solution de culture cellulaire pendant l'induction de différentiation de cellules souches pluripotentes et/ou après l'induction de différentiation ; et
l'évaluation d'un état de différenciation des cellules dans la solution de culture cellulaire sur la base d'une valeur de mesure de miARN,
dans lequel la fraction en phase liquide d'une solution de culture cellulaire est obtenue par séparation des cellules de la solution de culture cellulaire.

2. Procédé selon la revendication 1, dans lequel les cellules souches pluripotentes sont des cellules IPS, des cellules ES, des cellules NtES non humaines ou des cellules EG.

3. Procédé selon l'une quelconque des revendications 1 ou 2 dans lequel la valeur de mesure de miARN est une valeur de mesure de miR302a, une valeur de mesure de miR302b, une valeur de mesure de miR302c, une valeur de mesure de miR302d, une valeur de mesure de miR367, ou une somme d'au moins deux des valeurs de mesure.

4. Procédé selon l'une quelconque des revendications 1 à 3, dans lequel la valeur de mesure est obtenue par RT-PCR quantitative.

5. Procédé selon l'une quelconque des revendications 1 à 4, dans lequel
la valeur de mesure de miARN est comparée à un seuil prédéterminé,
lorsque la valeur de mesure de miARN n'est pas inférieure au seuil, il est déterminé que des cellules souches pluripotentes sont contenues dans la solution de culture cellulaire, et
lorsque la valeur de mesure de miARN est inférieure au seuil, il est déterminé qu'aucune cellule souche pluripotente n'est contenue dans la solution de culture cellulaire.

6. Procédé selon l'une quelconque des revendications 1 à 5, dans lequel le nombre de cellules souches pluripotentes dans la solution de culture cellulaire est calculé sur la base de la valeur de mesure de miARN.

7. Procédé selon l'une quelconque des revendications 1 à 6, dans lequel la mesure de miARN comprend : une mesure de miARN à un premier instant pendant l'induction de différenciation des cellules souches pluripotentes ; et une mesure de miARN à un deuxième instant pendant l'induction de différenciation des cellules souches pluripotentes.

8. Procédé de détection de cellules souches pluripotentes, comprenant les étapes de :
mesure d'au moins un miARN dans un cluster miR302/367 dans une fraction en phase liquide d'une solution de culture cellulaire pendant l'induction de différentiation de cellules souches pluripotentes et/ou après l'induction de différentiation ; et
la détection de cellules souches pluripotentes dans la solution de culture cellulaire sur la base d'une valeur de mesure de miARN,
dans lequel la fraction en phase liquide d'une solution de culture cellulaire est obtenue par séparation des cellules de la solution de culture cellulaire.

9. Procédé selon l'une quelconque des revendications 1 à 8, dans lequel les cellules souches pluripotentes sont des cellules qui ont été préparées par reprogrammation de cellules différenciées prélevées à partir d'un organisme.

10. Procédé selon l'une quelconque des revendications 1 à 9, dans lequel les miARN sont des ARN décrits dans SEQ ID NO : 1 à 5.
